# EUROPÄISCHE PATENTSCHRIFT

(11) **EP 1 210 928 B1**
(45) Veröffentlichungstag und Bekanntmachung des Hinweises auf die Patenterteilung: **21.09.2005**
(21) Anmeldenummer: 01127503.9
(22) Anmeldetag: 17.11.2001
(51) Int. Cl.: A61K 7/00, A61K 7/48, A61K 7/50

(54) **Kosmetische oder dermatologische getränkte Tücher**
Cosmetically or dermatologically impregnated towelettes
Serviettes cosmetiques et dermatologiques imbibées

(30) Priorität: 30.11.2000 DE 10059584
(43) Veröffentlichungstag der Anmeldung: 05.06.2002
(73) Patentinhaber: Beiersdorf Aktiengesellschaft, 20245 Hamburg (DE)
(72) Erfinder: Küther, Jörg, Dr., 25421 Schenefeld (DE); von der Fecht, Stephanie, 22869 Schenefeld (DE); Drucks, Anja, 22399 Hamburg (DE)

(56) Entgegenhaltungen:
- EP-A- 0 613 675
- WO-A-99/25318
- DE-A- 19 715 385
- DE-A- 19 903 903
- US-A- 5 635 191

## Beschreibung

Die vorliegende Erfindung betrifft oberflächenstrukturierte kosmetische und dermatologische Tücher, welche mit dünnflüssigen kosmetischen und dermatologischen Tränkungslösungen befeuchtet sind. Insbesondere betrifft die Erfindung kosmetische und dermatologische getränkte Pflege-, Reinigungs- und Deotücher sowie getränkte Tücher zur Bekämpfung von Hautkrankheiten (wie Akne etc.) und solche, welche die Haut nach einem Sonnenbad gezielt pflegen und die Nachreaktionen der Haut auf die Einwirkung von UV-Strahlung vermindern.

Getränkte Tücher finden als Gegenstände des täglichen Bedarfs breiten Einsatz in unterschiedlichsten Bereichen. Sie erlauben unter anderem effiziente und hautschonende Reinigung und Pflege besonders auch in der Abwesenheit von (fließendem) Wasser. Dabei besteht der eigentliche Gebrauchsgegenstand aus zwei Komponenten:
a) einem trockenen Tuch, welches aus Materialien wie Papier und/oder unterschiedlichsten Mischungen aus Natur- oder Kunstfasern aufgebaut ist und
b) einer niederviskosen Tränkungslösung.

Auch oberflächenstrukturierte Tücher sind an sich bekannt. Sie werden auf der Basis von Cellulose hergestellt und finden insbesondere als Haushaltstücher und zur perianalen Reinigung Verwendung. Ihre Struktur wird durch mechanische Prägung mittels Kalanderwalzen erzeugt. Derartige Tücher haben eine geringe Reißfestigkeit bei gleichzeitig großer Rauhigkeit und Härte. Sie eignen sich daher nur bedingt zur Verwendung an der menschlichen Haut.

Die WO-99/25318-A1 beschreibt ein Reinigungstuch für den persönlichen Gebrauch, umfassend ein einschichtiges Substrat, welches aus hydroverknäuelten Fasern gebildet wird, wobei das Substrat auf einem wesentlichen Bereich seiner Grundfläche ein dreidimensionales Muster aufweist. Dieses Substrat ist mit einer wässrigen flüssigen Reinigungszusammensetzung getränkt, welche eine wirksame Menge eines Reinigungstensids enthält, wobei der Tränkungsgrad des Tuches 100 bis 400 Gew.-% des Substrats beträgt.

Die EP-0 613 675-A1 beschreibt ein Reinigungstuch umfassend ein poröses oder absorbierendes Tuch, welches mit einer Öl-in-Wasser-Emulsion imprägniert ist, wobei die Emulsion mindestens ein antimikrobielles Agens enthält.

Die DE-197 15 385-A1 beschreibt Baby-Ölpflege- und -reinigungstücher mit einer Tränkung aus pflanzlichen Ölen.

Aufgabe der vorliegenden Erfindung war es daher, kosmetische oder dermatologische getränkte Tücher zu finden, die die Nachteile des Standes der Technik nicht zeigen und sich insbesondere zur Pflege und/oder Reinigung der Haut eignen.

Es war überraschend und für den Fachmann nicht vorauszusehen, daß kosmetische und dermatologische Tücher, wobei die Tücher aus einem wasserstrahlverfestigten und wasserstrahlgeprägten Vlies bestehen, welche mit kosmetischen und dermatologischen Tränkungslösungen befeuchtet sind, die eine Viskosität von weniger als 2000 mPa·s (Meßgerät: Haake Viskotester VT-02 bei 25 °C) aufweisen, dadurch gekennzeichnet, daß die Dicke des geprägten Vlieses mit den durch die Prägung erzeugten Erhebungen zwischen 5 % und 50 % größer ist als die Dicke des ungeprägten Vlieses und eine glechmäßige Abfolge von Erhebungen und Senken im Material entsteht, den Nachteilen des Standes der Technik abhelfen.

Die erfindungsgemäßen Tücher stellen die Kombination eines weichen, wasserunlöslichen, neuartig strukturierten Vliesgewebes (Nonwoven material) mit dünnflüssigen kosmetischen und dermatologischen Tränkungslösungen dar. Sie sind jeglicher Hinsicht überaus befriedigend und eignen sich dementsprechend ganz besonders, um als Grundlage für Zubereitungsformen mit vielfältigen Anwendungszwecken zu dienen. Die erfindungsgemäßen Tücher zeigen sehr gute sensorische und kosmetische Eigenschaften und zeichnen sich ferner durch hervorragende Hautpflegedaten aus.

Das Vliesgewebe wird vorzugsweise als Spunlace-Material im Herstellungsprozeß durch Wasserstrahlen verfestigt. Die Strukturierung erfolgt dann vorteilhaft ebenfalls durch Wasserstrahlen. Durch diese Strukturierung entsteht eine gleichmäßige Abfolge von Erhebungen und Senken im Material. In Kombination mit geeigneten Tränkungslösungen ermöglicht diese Strukturierung durch ihre Erhebungen sowohl einen besseren Zugang zu Vertiefungen in der menschlichen Haut als auch durch ihre Strukturtäler eine erhöhte Schmutzaufnahmekapazität. Dies führt insgesamt zu einer deutlich verbesserten Reinigungsleistung.
Ein besserer Zugang zu Vertiefungen in der menschlichen Haut ist zudem zur Bekämpfung von Hautkrankheiten und Hautirritationen sowie zur wirksamen Entfaltung einer desodorierenden Wirkung von besonderer Bedeutung.

Die kosmetischen und dermatologischen Tränkungslösungen, mit welchen die erfindungsgemäßen Tücher befeuchtet sind, können in verschiedenen Formen vorliegen. Sie sind vorzugsweise dünnflüssig, insbesondere sprühbar und haben z. B. eine Viskosität von weniger als 2000 mPa·s, insbesondere weniger als 1.500 mPa·s (Meßgerät: Haake Viskotester VT-02 bei 25 °C).

Die Tränkungslösungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch eine Mikroemulsion, eine Pickering-Emulsion, eine sprühbare Emulsion oder eine Hydrodispersion sein.

Darüberhinaus können die erfindungsgemäßen Formulierungen aber auch vorteilhaft in Form von ölfreien Zubereitungen - wie beispielsweise als Gele oder (wäßrige, alkoholische, wäßrig-alkoholische) Lösungen - vorliegen.

Sofern die Tränkungslösung im Sinne der vorliegenden Erfindung eine Lösung oder Dispersion darstellt, können als Lösungsmittel verwendet werden:
■ Wasser oder wäßrige Lösungen
■ Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die Tränkungslösung eine oder mehrere Wasserphasen enthält, können diese vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte sowie Polymere, Schaumstabilisatoren, Elektrolyte, Zuckerderivate und/oder Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Homschicht (auch transepidermal water loss (TEWL) genannt) zu reduzieren und/oder die Hydratation der Homschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registratumummer 178463-23-5 abgelegt und z. B. unter der Bezeichnung Fucogel®1000 von der Gesellschaft SOLABIA S.A. erhältlich ist.

Ferner vorteilhaft im Sinne der vorliegenden Erfindung sind wasserfreie Zubereitungen, welche neben einer oder mehreren Ölkomponenten weitere öllösliche Hilfs-, Zusatz- und/oder Wirkstoffe enthalten können.

Sofern die Tränkungslösung eine oder mehrere Ölphasen enthält, werden das oder die Öle im Sinne der vorliegenden Erfindung vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner können die Öle vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft werden die Öle gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wodurch beispielsweise W/S-(Wasser-in-Silikon-), S/W- (Silikon-in-Wasser-) Formulierungen und dergleichen mehr entstehen. Es wird allerdings bevorzugt, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Das oder die Öle werden ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 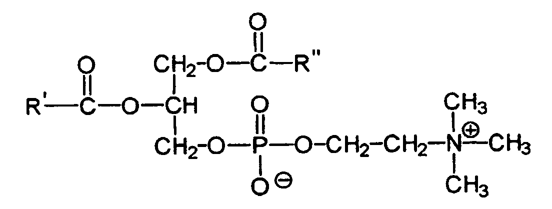 auszeichnen, wobei R' und R" typischerweise unverzweigte aliphatische Reste mit 15 oder 17 Kohlenstoffatomen und bis zu 4 cis-Doppelbindungen darstellen.

Die Tücher im Sinne der vorliegenden Erfindung enthalten vorteilhaft eines oder mehrere waschaktive Tenside der folgenden vier Gruppen A bis D, insbesondere wenn sie als Reinigungstücher verwendet werden sollen:

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,

Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10 Phosphat und Dilaureth-4 Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Vorteilhaft zu verwendende kationische Tenside sind
1. Alkylamine,
2. Alkylimidazole,
3. Ethoxylierte Amine und
4. Quarternäre Tenside.
Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind Benzalkoniumchlorid, Alkylbetain, Alkylamidopropylbetain und Alkyl-amidopropylhydroxysultain.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Die Tränkungslösungen enthalten besonders vorteilhaft eines oder mehrere waschaktive Tenside aus den Gruppe der Tenside, welchen einen HLB-Wert von mehr als 25 haben, ganz besonders solche, welchen einen HLB-Wert von mehr als 35 haben.

Es ist vorteilhaft im Sinn der vorliegenden Erfindung, wenn der Gehalt an einem oder mehreren waschaktiven Tensiden in der kosmetischen oder dermatologischen Tränkungslösung aus dem Bereich von 5 bis 25 Gew.-%, ganz besonders vorteilhaft von 10 bis 15 Gew.-% gewählt wird, jeweils bezogen auf das Gesamtgewicht der Tränkungslösung.

Die Tränkungslösungen für die erfindungsgemäßen kosmetischen und dermatologischen Tücher enthalten weiterhin vorteilhaft auch Konservierungsmittel.

Konservierungsmittel sind antimikrobielle Substanzen, die beim Herstellungsprozeß einem Produkt (Nahrungs- oder Genußmittel, pharmazeutische, kosmetische oder auch chemisch-technische Zubereitungen) in geringen Mengen (gewöhnlich je nach Produkt zwischen ca. 0,0005 % und 1 % Aktivgehalt) zugesetzt werden. Konservierungsmittel sollen Produkte während der Herstellung, der Lagerung und des Gebrauchs vor Verunreinigungen durch Mikroorganismen insbesondere vor den mikrobiell bedingten nachteiligen Veränderungen schützen.

An ein Konservierungsmittel werden grundsätzlich nachfolgende Forderungen gestellt: Es muß ausreichend antimikrobiell wirksam, technologisch anwendbar und gesundheitlich unbedenklich sein. Die gesundheitliche Unbedenklichkeit muß aber auch die fertige Zubereitung, das Handelsprodukt erfüllen. Dabei ist zu berücksichtigen, daß Mikroorganismen in z. B. kosmetischen Mitteln primär produktionsbedingt vorhanden sein oder sekundär durch den Verbraucher in das kosmetische Mittel gelangen können.

Daher muß gewährleistet sein, daß das Fertigprodukt auch über den gesamten Verbrauchszeitraum sicher ist.

Die meisten für eine Konservierung vorgeschlagenen bzw. vorgesehenen Konservierungsmittel wirken bakteriostatisch und fungistatisch, gelegentlich auch bakterizid und fungizid: sie sollen geruch- und geschmacklos und in den zur Anwendung kommenden Dosen nach Möglichkeit löslich, nicht toxisch, hautverträglich und ausreichend wirksam sein. Die Konservierungsmittel müssen, um wirksam zu sein, in dem zu konservierenden Roh- oder Hilfsstoff gelöst sein. Da die meisten Konservierungsmittel besser fett- als wasserlöslich sind, muß damit gerechnet werden, daß z. B. in einer Emulsion, deren wäßrige Phase konserviert werden soll, das in die wäßrige Phase eingearbeitete Konservierungsmittel im Verlauf der Lagerung in die Fettphase auswandert und damit die Konservierung der wäßrigen Phase in Frage gestellt ist. Aus diesem Grunde empfiehlt es sich, eine Kombination von Konservierungsmitteln einzusetzen, d. h. die wäßrige Phase mit einem gut wasserlöslichen Konservierungsmittel, die Fettphase dagegen gleichzeitig mit einem fettlöslichen Konservierungsmittel zu konservieren.

Für ein kosmetisches Präparat braucht zwar in der Regel keine Sterilität gefordert zu werden, es muß aber frei von pathogenen Keimen sein und vor mikrobiell bedingten Veränderungen geschützt werden.

Man sollte berücksichtigen, daß verschiedene Emulsionstypen, wäßrige Lösungen, Suspensionen usw. eine unterschiedliche Konservierung brauchen, daß die konservierende Wirkung einzelner Konservierungsmittel von der Zusammensetzung und den physikalischen Eigenschaften der zu konservierenden Zubereitung abhängig ist, daß mit Interaktionen zwischen dem Konservierungsmittel, den Wirk- und Hilfsstoffen zu rechnen ist, daß verschiedene Wirk- oder Hilfsstoffe Konservierungsmittel adsorbieren und damit möglicherweise inaktivieren können, daß insbesondere in der Zubereitung enthaltene Hydrokolloide konzentrationsabhängig Konservierungsmittel in ihrer antimikrobiellen Aktivität behindern können und daß schließlich - wiederum in Abhängigkeit von der Konzentration und dem Typ des Konservierungsmittels - das Stratum corneum das Konservierungsmittel adsorbiert und das Konservierungsmittel dann möglicherweise zur Permeation und Absorption kommt.

In der Lebensmitteltechnologie zugelassene Konservierungsmittel, welche auch vorteilhaft im Sinne der vorliegenden Erfindung eingesetzt werden können, sind mit ihrer E-Nummer nachfolgend aufgeführt.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | -Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol.

Besonders vorteilhafte kosmetische Tränkungslösungen im Sinne der vorliegenden Erfindung erhalten ferner Antioxidantien als Zusatz- oder Wirkstoffe. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden.

Bevorzugte Wirkstoffe sind Antioxidantien, welche die Haut vor oxidativer Beanspruchung schützen können. Besonders bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkemöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen. Bevorzugter NO-Synthasehemmer ist das Nitroarginin.

Getränkte Tücher im Sinne der vorliegenden Erfindung eignen sich dementsprechend insbesondere vorteilhaft zur Prophylaxe und Behandlung kosmetischer oder dermatologischer Hautveränderungen, wie sie z. B. bei der Hautalterung auftreten. Weiterhin vorteilhaft eignen sie sich gegen das Erscheinungsbild der trockenen bzw. rauhen Haut.

Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:
a) Trockenheit, Rauhigkeit und Ausbildung von (Trockenheits-) Fältchen,
b) Juckreiz und
c) verminderte Rückfettung durch Talgdrüsen (z. B. nach dem Waschen).
   Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

In einer besonderen Ausführungsform betrifft die vorliegende Erfindung insbesondere Produkte zur Pflege der auf natürliche Weise gealterten Haut, sowie zur Behandlung der Folgeschäden der Lichtalterung, insbesondere der unter a) bis g) aufgeführten Phänomene.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

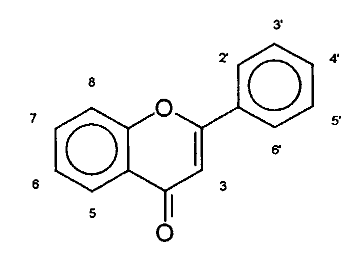

Einige der wichtigeren Flavone, welche auch bevorzugt in erfindungsgemäßen Tränkungslösungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt gewählt aus der Gruppe der Substanzen der generischen Strukturformel 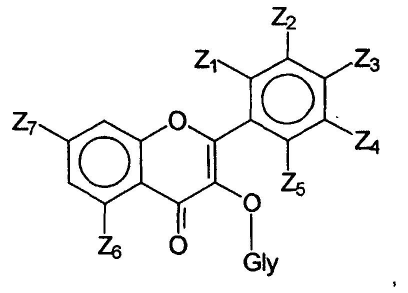 wobei Z₁ bis Z₇ unabhängig voneinander gewählt werden aus der Gruppe H, OH, Alkoxy- sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 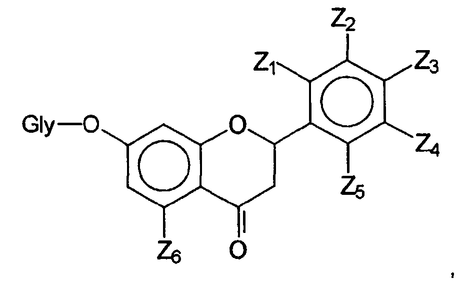 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 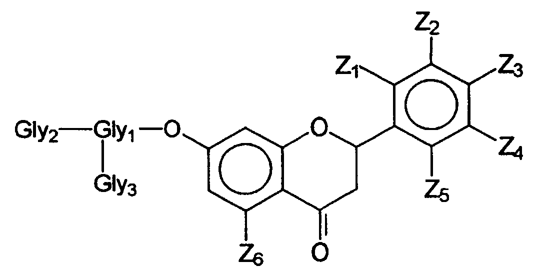 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

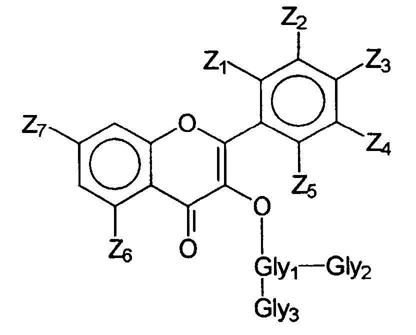

Besonders vorteilhaft werden die erfindungsgemäßen Flavonglycoside aus der Gruppe, welche durch die folgende Struktur wiedergegeben werden: 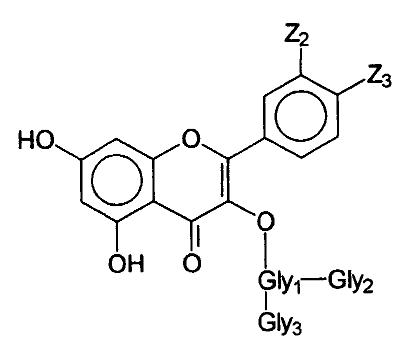 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel 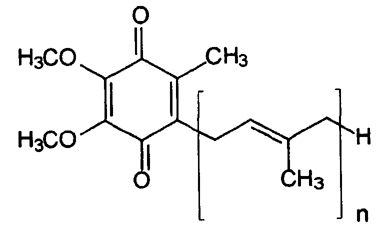 aus und stellen die am weitesten verbreiteten u. damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen u. Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

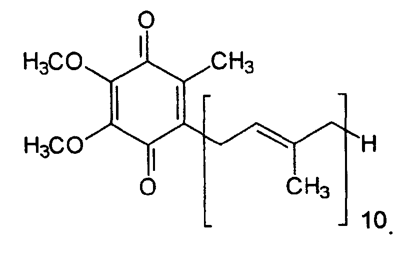

Plastochinone weisen die allgemeine Strukturformel 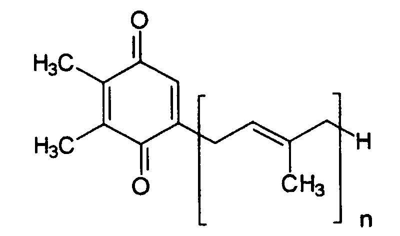 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

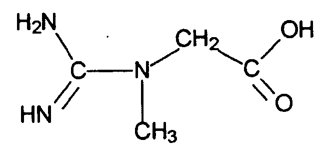

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-buttersäurebetain]. Auch Acyl-Camitine, welche gewählt aus der Gruppe der Substanzen der folgenden allgemeinen Strukturformel 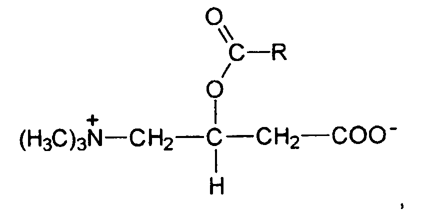 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcamitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Die Liste der genannten Wirkstoffe bzw. Wirkstoffkombinationen, die in den erfindungsgemäßen Tränkungslösungen verwendet werden können, soll selbstverständlich nicht limitierend sein. Die Wirkstoffe können einzelnen oder in beliebigen Kombinationen miteinander verwendet werden.

Günstig sind solche kosmetischen und dermatologischen Tücher, die in der Form eines Sonnenschutzmittels vorliegen. Es ist aber auch vorteilhaft im Sinne der vorliegenden Erfindungen, solche kosmetischen und dermatologischen Tücher zu erstellen, deren hauptsächlicher Anwendungszweck nicht der Schutz vor Sonnenlicht ist, die aber dennoch einen Gehalt an UV-Schutzsubstanzen enthalten.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Dementsprechend enthalten die Tränkungslösungen im Sinne der vorliegenden Erfindung vorzugsweise neben einer oder mehreren erfindungsgemäßen UV-Filtersubstanzen zusätzlich mindestens eine weitere UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden.

Solche Pigmente können im Sinne der vorliegenden Erfindung vorteilhaft oberflächlich behandelt ("gecoatet") sein, wobei beispielsweise ein amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Erfindungsgemäß vorteilhaft sind z. B. Titandioxidpigmente, die mit Octylsilanol beschichtet sind. Geeignete Titandioxidpartikel sind unter der Handelsbezeichnung T805 bei der Firma Degussa erhältlich. Besonders vorteilhaft sind ferner mit Aluminiumstearat beschichtete TiO₂-Pigmente, z. B. die unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 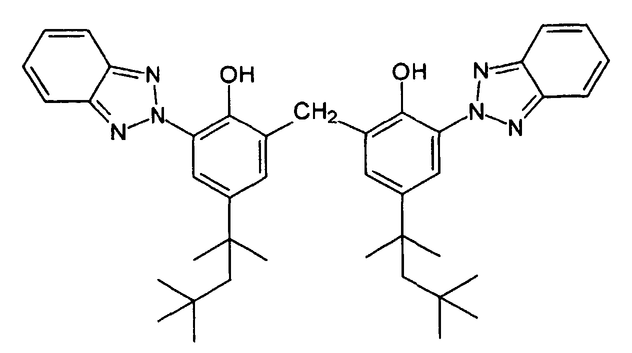 gekennzeichnet ist und unter der Handelsbezeichnung Tinosorb® M bei der CIBA-Chemikalien GmbH erhältlich ist.

Vorteilhaft enthalten erfindungsgemäße Tränkungslösungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Tränkungslösungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate, welches beispielsweise unter der Handelsbezeichnung Neo Heliopan AP bei Haarmann&Reimer erhältlich ist sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Besonders bevorzugte UV-Filtersubstanz im Sinne der vorliegenden Erfindung ist ferner ein unsymmetrisch substituiertes s-Triazin, welches auch als Dioctylbutylamidotriazon (INCI: Dioctylbutamidotriazone) bezeichnet wird und unter der Handelsbezeichnung UVA-SORB HEB bei Sigma 3V erhältlich ist.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-B-Filter können öllöslich oder wasserlöslich sein. Vorteilhafte öllösliche UV-B-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Es kann auch von erheblichem Vorteil sein, polymergebundene oder polymere UV-Filtersubstanzen in Tränkungslösungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in die kosmetischen oder dermatologischen Tränkungslösungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Die erfindungsgemäßen kosmetischen und dermatologischen Tücher können ferner vorteilhaft Farbstoffe und/oder Farbpigmente enthalten, insbesondere wenn sie im Bereich der dekorativen Kosmetik verwendet werden sollen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo-1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)Δ^{2,5}-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Periglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂ · 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Sofern die erfindungsgemäßen Tücher für die Anwendung im Gesichtsbereich vorgesehen sind, ist es günstig, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner getränkte Tücher mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
2. Monokristalline Perlglanzpigmente wie z. B. Bismuthoxychlorid (BiOCI)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung / Schichtdicke** | **Farbe** |
|---|---|---|
| **Silberweiße Perlglanzpigmente** | TiO₂: 40 - 60 nm | silber |
| **Interferenzpigmente** | TiO₂: 60 - 80 nm | gelb |
| | TiO₂: 80 - 100 nm | rot |
| | TiO₂: 100 - 140 nm | blau |
| | TiO₂: 120 - 160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Tränkungslösungen.

Vorteilhaft im Sinne der vorliegenden Erfindung sind ferner Tücher, welche als kosmetische oder dermatologische Deo- bzw. Antitranspirant-Tücher Anwendung finden.

Als erfindungsgemäß besonders vorteilhaft enthalten diese einen oder mehrere der üblichen desodorierenden und/oder antitranspirant wirksamen Wirkstoffe, beispielsweise Geruchsüberdecker, wie die gängigen Parfümbestandteile, Geruchsabsorber, beispielsweise die in der Patentoffenlegungschrift DE-P 40 09 347 beschriebenen Schichtsilikate, von diesen insbesondere Montmorillonit, Kaolinit, Nontronit, Saponit, Hectorit, Bentonit, Smectit, ferner beispielsweise Zinksalze der Ricinolsäure. Keinhemmende Mittel sind ebenfalls geeignet, in die erfindungsgemäßen Zubereitungen eingearbeitet zu werden. Vorteilhafte Substanzen sind 2,4,4'-Trichlor-2'hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-37 40 186, DE-39 38 140, DE-42 04 321, DE-42 29 707, DE-42 29 737, DE-42 37 081, DE- 43 09 372, DE-43 24 219 beschriebenen wirksamen Agenzien.

Die üblichen Antitranspiranswirkstoffe können ebenfalls vorteilhaft verwendet werden, beispielsweise Aluminiumchlorid, Aluminiumchlorhydrat, -nitrat, -sulfat, -acetat usw. Ferner vorteilhaft sind auch Zink-, Magnesium- und Zirkoniumverbindungen. Übliche und vorzugsweise zu verwendende Antitranspiranswirkstoffe sind beispielsweise beschrieben in: *H. P. Fiedler, Der Schweiß, Editio Cantor, Aulendorf, 2. Auflage, S. 303-377, Kapitel K: "Mittel zur Hemmung der Transpiration"*.

Es ist auch vorteilhaft im Sinne der vorliegenden Erfindung, solche kosmetischen und dermatologischen Tücher zu erstellen, deren hauptsächlicher Zweck nicht die Deo- bzw. Antitranspirantwirkung ist, die aber dennoch einen Gehalt an üblichen desodorierenden und/oder antitranspirant wirksamen Wirkstoffen aufweisen.

Die erfindungsgemäßen Tücher eignen sich ferner hervorragend als Träger für dermatologische Wirkstoffe, z. B. als Träger für gegen Akne wirksame Substanzen. Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgaris sind die Verhomung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien (*Propionibacterium acnes*).

Daher ist es vorteilhaft, den erfindungsgemäßen Tränkungslösungen gegen Akne wirksame Substanzen zuzugeben, die beispielsweise gegen *Propionibacterium acnes* wirksam sind (etwa solche, die in DE-OS 42 29 707, DE-OS 43 05 069, DE-OS 43 07 976, DE-OS 43 37 711, DE-OS 43 29 379 beschrieben werden) aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol (α-Octadecylglycerylether), Selachylalkohol (α-9-Octadecenylglycerylether), Chimylalkohol (α-Hexadecylglycerylether) und/oder Bisabolol sowie Antibiotika und/oder Keratolytika.

Keratolytika sind Stoffe, die verhornte Haut (wie z. B. Warzen, Hühneraugen, Schwielen und dergleichen mehr) erweichen, damit sich diese leichter entfernen läßt oder damit sie abfällt bzw. sich auflöst.

Alle gängigen gegen Akne wirksamen Substanzen können vorteilhaft genutzt werden, insbesondere Benzoylperoxid, Bituminosulfonate (Ammonium-, Natrium- und CalciumSalze von Schieferöl-Sulfonsäuren), Salicylsäure (2-Hydroxybenzoesäure), Miconazol (1-[2-(2,4-Dichlorbenzyloxy)-2-(2,4-dichlorphenyl)-ethyl]-imidazol) und Derivate, Adapalen (6-[3-(1-Adamantyl)-4-methoxyphenyl]-2-naphthoesäure), Azelainsäure (Nonandisäure), Mesulfen (2,7--Dimethylthianthren, C₁₄H₁₂S₂) sowie Aluminiumoxid, Zinkoxid und/oder feinverteilter Schwefel.

Die Menge der Antiaknemittel (eine oder mehrere Verbindungen) in den Tränkungslösungen beträgt vorzugsweise 0,01 bis 30 Gew.-%, besonders bevorzugt 0,1 bis 20 Gew.-%, insbesondere 1 bis 10 Gew.-%, bezogen auf das Gesamtgewicht der Tränkungslösung.

Erfindungsgemäß werden in Kombination mit den dünnflüssigen kosmetischen und dermatologischen Tränkungslösungen Tücher eingesetzt, die aus einem insbesondere wasserstrahlverfestigten und wasserstrahlgeprägten Vlies (Spunlaced-Material) bestehen.

Die in das Vlies eingebrachte Makroprägung kann jedes gewünschte Muster aufweisen. Die zu treffende Auswahl richtet sich nach zum einen nach der aufzubringenden Tränkung und zum anderen nach dem Einsatzfeld, auf dem das spätere Tuch Verwendung finden soll.

Große Kavitäten an der Vliesoberfläche und im Vlies erleichtem die Aufnahme von Schmutz und Verunreinigungen, wenn mit dem getränkten Tuch über die Haut gefahren wird. Die Reinigungswirkung wird gegenüber den ungeprägten Tüchern um ein Vielfaches gesteigert.

Die Dicke des geprägten Vlieses mit den durch die Prägung erzeugten Erhebungen ist zwischen 5 % und 50 % größer als die Dicke des ungeprägten Vlieses. Vorteilhafterweise ist die Dicke des Vlieses mit den durch die Prägung erzeugten Erhebungen ungefähr doppelt so groß wie die des ungeprägten Vlieses. In bevorzugten Ausführungsformen ist das geprägte Vlies zwischen 10 % und 25 % dicker als das ungeprägte.

Das geprägte Vlies weist des weiteren besondere Eigenschaften auf, die die Verwendung als Trägermaterial für Emulsionen oder sonstigen Zubereitungen ermöglichen.

So beträgt die Reißkraft insbesondere

| | | [N/50mm] |
|---|---|---|
| im trockenen Zustand | Maschinenrichtung | >60, vorzugsweise >80 |
| | Querrichtung | >20, vorzugsweise >30 |
| im getränkten Zustand | Maschinenrichtung | >4, vorzugsweise >60 |
| | Querrichtung | >10, vorzugsweise >20 |

Die Dehnfähigkeit des Tuches beträgt vorzugsweise

| | | |
|---|---|---|
| im trockenen Zustand | Maschinenrichtung | 15 % bis 100 %, bevorzugt |
| | | 20 % und 50 % |
| | Querrichtung | 40 % bis 120 %, bevorzugt |
| | | 50 % und 85 % |
| im getränkten Zustand | Maschinenrichtung | 15 % bis 100 %, bevorzugt |
| | | 20 % und 40 % |
| | Querrichtung | 40 % bis 120 %, bevorzugt |
| | | 50 % und 85 % |

Es hat sich als vorteilhaft herausgestellt für das Tuch, wenn dieses ein Gewicht von 35 bis 120 g/m², vorzugsweise von 40 bis 60 g/m², hat (gemessen bei 20 °C ± 2 °C und bei einer Feuchtigkeit der Raumluft von 65 % ± 5 % für 24 Stunden).

Die Dicke des Vlieses beträgt vorzugsweise 0,4 mm bis 1,5 mm, insbesondere 0,6 mm bis 0,9 mm.

Schließlich ist es für das Tuch besonders vorteilhaft, ein "surface linting" von weniger als 4 mg/1000mm², vorzugsweise weniger als 2 mg/1000mm² zu haben.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasem angeführt. Die vorliegende Erfindung ist aber nicht auf die genannten Materialien beschränkt, sondern es können eine Vielzahl weiterer Fasern zur Vliesbildung eingesetzt werden.

In einer besonders vorteilhaften Ausführungsform des Vlieses bestehen die Fasern aus einer Mischung aus 70 % Viskose und 30 % PET.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Sodann weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Die nachfolgenden Beispiele sollen die erfindungsgemäßen Tränkungslösungen verdeutlichen. Die Zahlenwerte in den Beispielen bedeuten Gewichtsprozente, bezogen auf das Gesamtgewicht der jeweiligen Zubereitungen.

### Beispiele:

### Beispiel 1

| **Bestandteil** | **Menge / Gew.-%** |
|---|---|
| Paraffinum Liquidum | 99,8 |
| Parfum | 0,2 |

### Beispiel 2: Mikroemulsion

| **Bestandteil** | **Menge / Gew.-%** |
|---|---|
| Wasser | 82,0 |
| Paraffinum Liquidum | 8,0 |
| Glycerin | 5,0 |
| Octylstearat | 2,0 |
| Glyceryl Stearate, Ceteareth-20, Ceteareth-12, Cetearyl Alcohol, Cetyl Palmitate | 1,5 |
| Phenoxyeheanol, Methylparaben, Ethylparaben, Propylparaben, Butylparaben, Isobutylparaben | 0,5 |
| Parfum | 0,4 |
| Ceteareth-20 | 0,3 |
| Methylparaben | 0,3 |
| Summe: | 100,0 |

### Beispiel 3: Mikroemulsion

| **Bestandteil** | **Menge / Gew.-%** |
|---|---|
| Wasser | 75,0 |
| Paraffinum Liquidum | 0,5 |
| Glycerin | 7,0 |
| Octylstearat | 1,0 |
| Glyceryl Stearate, Ceteareth-20, Ceteareth-12, Cetearyl Alcohol, Cetyl Palmitate | 3,0 |
| Phenoxyeheanol, Methylparaben, Ethylparaben, Propylparaben, Butylparaben, Isobutylparaben | 0,5 |
| Parfum | 2,0 |
| Ceteareth-20 | 10,0 |
| Methylparaben | 1,0 |
| Summe: | 100,0 |

### Beispiel 4: wäßrige Tränkungslösung

| **Bestandteil** | **Menge / Gew.-%** |
|---|---|
| Wasser | 96,89 |
| Butylenglykol | 1,0 |
| PEG-40 Hydrogenated Castor Oil | 0,8 |
| Phenoxyehanol, Methylparaben, Ethylparaben, Propylparaben, Butylparaben, Isobutylparaben | 0,65 |
| Kaliumsorbat | 0,3 |
| Parfum | 0,2 |
| Zitronensäure | 0,16 |
| Summe: | 100,0 |

### Beispiel 5: wäßrige Tränkungslösung

| **Bestandteil** | **Menge / Gew.-%** |
|---|---|
| Wasser | 95,0 |
| Butylenglykol | 1,0 |
| PEG-40 Hydrogenated Castor Oil | 1,0 |
| Phenoxyethanol,Methylparaben, Ethylparaben, Propylparaben, Butylparaben, Isobutylparaben | 1,5 |
| Kaliumsorbat | 0,5 |
| Parfum | 0,5 |
| Zitronensäure | 0,5 |
| Summe: | 100,0 |

### Beispiel 6

| **Bestandteil** | **Menge / Gew.-%** |
|---|---|
| Cyclomethicone | 65,5 |
| Dimethicone | 20,0 |
| Silikongum | 7,0 |
| Phenyltrimethylmethicone | 7,0 |
| Parfum | 0,5 |
| Summe: | 100,0 |

### Beispiel 7: alkoholische Tränkungslösung

| **Bestandteil** | **Menge / Gew.-%** |
|---|---|
| Ethanol | 60,0 |
| Wasser | 34,5 |
| Glycerin | 5,0 |
| Parfum | 0,5 |
| Summe: | 100,0 |

### Beispiel 8: alkoholische Tränkungslösung

| **Bestandteil** | **Menge / Gew.-%** |
|---|---|
| Ethanol | 60,0 |
| Wasser | 24,0 |
| Glycerin | 5,0 |
| Isopropylalkohol | 5,0 |
| Ethylenediamine | 1,0 |
| Dexpanthenol | 1,0 |
| Carbomer | 3,0 |
| Parfum | 0,5 |
| Farbstoff | 0,5 |
| Summe: | 100,0 |

### Beispiel 9: After Sun-/Hautpflege-Mikroemulsion

| **Bestandteil** | **Menge / Gew.-%** |
|---|---|
| Ceteth-15 | 6 |
| Glycerylisostearate | 2 |
| Cetyl Alkohol | 1 |
| Dicaprylyl Carbonate | 5 |
| Octyldodecanol | 3 |
| Cylomethicone | 1 |
| Butylene Glycol | 3 |
| Ethanol | 5 |
| DMDM Hydantoin | 0,6 |
| Octoxyglycerin | 1 |
| Antioxydantien | 0,5 |
| Parfuem, | 0,5 |
| Farbstoffe | 0,3 |
| Wasser | ad 100 |

### Beispiel 10: nicht fettende Körperpflegeemulsion

| **Bestandteil** | **Gew.-%** |
|---|---|
| Ceteareth-12 | 6 |
| Glyceryl Stearate | 3,5 |
| Cetyl Palmitate | 3 |
| Dicaprylyl Ether | 5 |
| Cycolmethicone | 3 |
| Phenyl Trimethicone | 1 |
| Paraffinwax | 2 |
| Glycerin | 7,5 |
| Parabene | 1 |
| Phenoxyethanol | 1 |
| AGR | 0,5 |
| Parfuem | 0,5 |
| Farbstoffe | 0,5 |
| Wasser | ad 100 |

### Beispiel 11: Sonnenschutzmittel für seidiges Hautgefühl

| **Bestandteil** | **Gew.-%** |
|---|---|
| Ceteareth-20 | 5,5 |
| Glyceryl Stearate | 4 |
| Stearyl Alkohol | 3 |
| Dicaprylyl Ether | 5 |
| Octyldodecanol | 3 |
| Phenyl Trimethicone | 1 |
| Bis-Ethylhexyloxyphenol Methoxyphenyl Triazine | 2 |
| Octocrylene | 7 |
| Diethylhexyl Butamido Triazone | 1 |
| Ethylhexyl Methoxycinnamate | 4 |
| Butylene Glycol | 1 |
| Vitamin E Acetat | 1 |
| PVP/Hexadecene Copolymer | 1 |
| Parabene | 1 |
| Antioxydantien | 0,5 |
| Parfuem | 0,5 |
| Wasser | ad 100 |

### Beispiel 12: Sonnenschutzformulierung

| **Bestandteil** | **Gew.-%** |
|---|---|
| Ceteareth-20 | 6,5 |
| Glyceryl Stearate | 2 |
| Stearyl Alkohol | 1 |
| Dicaprylyl Carbonate | 5 |
| Octyldodecanol | 3 |
| C12-15 Alkyl Benzoate | 1 |
| Titandioxid | 2 |
| Bis-Ethylhexyloxyphenol Methoxyphenyl Triazine | 2 |
| Octocrylene | 7 |
| Ethylhexyl Methoxycinnamate | 4 |
| Parabene | 1 |
| Antioxydantien | 0,5 |
| Parfuem | 0,5 |
| Wasser | ad 100 |

### Beispiel 13: Sonnenschutzformulierung

| **Bestandteil** | **Gew.-%** |
|---|---|
| Steareth-20 | 6,5 |
| Glycerylisostearate | 2 |
| Cetyl Alkohol | 1 |
| Dicaprylyl Carbonate | 5 |
| Shea Butter | 3 |
| C12-15 Alkyl Benzoate | 1 |
| Bis-Ethylhexyloxyphenol Methoxyphenyl Triazine | 2 |
| Butylmethoxydibenzoylmethane | 1 |
| Ethylhexyl Triazone | 2 |
| Phenylbenzimidazol Sulfonsäure | 2 |
| Ethylhexyl Methoxycinnamate | 4 |
| Glycerin | 10 |
| Tricontanyl PVP | 1 |
| Citrat-Puffer | 1 |
| Parabene | 1 |
| Antioxydantien | 0,5 |
| Parfuem | 0,5 |
| Wasser | ad 100 |

### Beispiel 14: Sonnenschutzformulierung

| **Bestandteil** | **Gew.-%** |
|---|---|
| Ceteareth-30 | 7 |
| Glycerylisostearate | 2,5 |
| Cetyl Alkohol | 1 |
| Dicaprylyl Carbonate | 4 |
| Capric/Caprylic Triglyceride | 2 |
| C12-15 Alkyl Benzoate | 6 |
| Methylen Bis-Benzotriazolyl Tetramethylbutylphenol | 2 |
| Butyl Methoxydibenzoylmethane | 2 |
| Ethylhexyl Triazone | 4 |
| Bis-Imidazylat | 2 |
| Methylbenzylidene Camphor | 4 |
| Glycerin | 5 |
| PVP Hexadecene Copolymer | 1 |
| Parabene | 1 |
| Antioxydantien | 0,5 |
| Parfuem | 0,5 |
| Wasser | ad 100 |

### Beispiel 15: Sonnenschutzformulierung

| **Bestandteil** | **Gew.-%** |
|---|---|
| Ceteareth-20 | 7,5 |
| Glyceryl Stearate | 3 |
| Cetyl Palmitate | 1,5 |
| Dicaprylyl Carbonate | 5 |
| Cocoglycerides | 2 |
| C12-15 Alkyl Benzoate | 6 |
| Barium Sulfate | 2 |
| Bis-Ethylhexyloxyphenol Methoxyphenyl Triazine | 2 |
| Ethylhexyl Triazone | 4 |
| Bis-Imidazylat | 1 |
| Phenylbenzimidazol Sulfonsäure | 2 |
| Methylbenzylidene Camphor | 4 |
| PVP Hexadecene Copolymer | 1 |
| NaOH | 0,5 |
| Parabene | 1 |
| Antioxydantien | 0.5 |
| Parfuem | 0,5 |
| Wasser | ad 100 |

### Beispiel 16: After Sun-/Hautpflege-Formulierung

| **Bestandteil** | **Gew.-%** |
|---|---|
| Ceteth-15 | 6 |
| Glycerylisostearate | 2 |
| Cetyl Alkohol | 1 |
| Dicaprylyl Carbonate | 5 |
| Shea Butter | 1 |
| Octyldodecanol | 3 |
| Cylomethicone | 1 |
| Mineral Oil | 2 |
| Ethanol | 5 |
| Parabene | 1 |
| Antioxydantien | 0,5 |
| Parfuem | 0,5 |
| Wasser | ad 100 |

### Öl - Beispiele

| **Öl-1** | |
|---|---|
| **Bestandteil** | **Gew.-%** |
| Capric/Caprylic Triglyceride | 2 |
| C12-15 Alkyl Benzoate | 6 |
| Butyl Methoxydibenzoylmethane | 2 |
| Ethylhexyl Triazone | 2 |
| Bis-Ethylhexyloxyphenol Methoxyphenyl Triazine | 1 |
| Methylbenzylidene Camphor | 4 |
| Shea Butter | 1 |
| Butylene Glycol Dicaprate/Dicaprylate | 3 |
| Dimethicone | 5 |
| Parabene | 1 |
| Antioxydantien | 0,5 |
| Parfuem | 0,5 |
| Mineraloil | ad 100 |

| **Öl-2** | |
|---|---|
| **Bestandteil** | **Gew.-%** |
| Dicaprylyl Carbonate | 5 |
| Bis-Ethylhexyloxyphenol Methoxyphenyl Triazine | 2 |
| Ethylhexyl Triazone | 4 |
| Methylbenzylidene Camphor | 4 |
| Shea Butter | 1 |
| Octyldodecanol | 3 |
| Cylomethicone | 1 |
| Vitamin E | 1 |
| Parfuem | 0,5 |
| Mineraloil | ad 100 |

| **Öl-3** | |
|---|---|
| **Bestandteil** | **Gew.-%** |
| Dicaprylyl Carbonate | 5 |
| Bis-Ethylhexyloxyphenol Methoxyphenyl Triazine | 2 |
| Diethylhexyl Butamido Triazone | 4 |
| Methylbenzylidene Camphor | 1 |
| Shea Butter | 1 |
| Phenyltrimethicone | 1 |
| Vitamin E | 2 |
| Parfuem | 0,5 |
| Cyclomethicone | ad 100 |

| **Öl-4** | |
|---|---|
| **Bestandteil** | **Gew.-%** |
| Ethylhexyl Methoxycinnamate | 10 |
| Dicaprylyl Carbonate | 5 |
| Bis-Ethylhexyloxyphenol Methoxyphenyl Triazine | 2 |
| Diethylhexyl Butamido Triazone | 4 |
| Octocrylene | 5 |
| Shea Butter | 1 |
| Phenyltrimethicone | 1 |
| Vitamin E | 1 |
| Parfuem | 1 |
| Cyclomethicone | ad 100 |

| **Wäßrige Formulierung:** | |
|---|---|
| **Bestandteil** | **Gew.-%** |
| Bis-Imidazylat | 1 |
| Phenylbenzimidazol Sulfonsäure | 2 |
| Glycerin | 10 |
| Parabene | 1 |
| Antioxydantien | 0,5 |
| Parfuem | 0,5 |
| Wasser | ad 100 |

## Patentansprüche

1. Kosmetische und dermatologische Tücher, wobei die Tücher aus einem wasserstrahlverfestigten und wasserstrahlgeprägten Vlies bestehen, welche mit kosmetischen und dermatologischen Tränkungslösungen befeuchtet sind, die eine Viskosität von weniger als 2000 mPa·s (Meßgerät: Haake Viskotester VT-02 bei 25 °C) aufweisen, **dadurch gekennzeichnet, daß** die Dicke des geprägten Vlieses mit den durch die Prägung erzeugten Erhebungen zwischen 5 % und 50 % größer ist als die Dicke des ungeprägten Vlieses und eine gleichmäßige Abfolge von Erhebungen und Senken im Material entsteht.

2. Tücher nach Anspruch 1, **dadurch gekennzeichnet, daß** die Dicke des geprägten Vlieses mit den durch die Prägung erzeugten Erhebungen zwischen 10 % und 25 % größer ist als die Dicke des ungeprägten Vlieses.

3. Tücher nach einem der vorhergehenden Ansprüche, **dadurch gekennzeichnet, daß** das Gewichtsverhältnis des ungetränkten Tuchs zu der Tränkungslösung aus dem Bereich von 1 : 1 bis 1 : 5 gewählt wird.

4. Tücher nach einem der vorhergehenden Ansprüche, **dadurch gekennzeichnet, daß** die Tränkungslösung aus Ölen einschließlich Silikonölen und/oder lipohilen Substanzen besteht und/oder weniger als 0,5 Gew.-% Wasser - bezogen auf das Gesamtgewicht der Tränkungslösung - enthält.

5. Tücher nach einem der vorhergehenden Ansprüche, **dadurch gekennzeichnet, daß** die Tränkungslösung neben einer oder mehrerer Ölphasen zusätzlich eine oder mehrere Wasserphasen enthält und in Form einer W/O-, O/W-, W/O/W- oder O/W/O-Emulsion, einer Mikroemulsion, einer Pickering-Emulsion, einer sprühbare Emulsion oder einer Hydrodispersion vorliegt.

6. Tücher nach einem der vorhergehenden Ansprüche, **dadurch gekennzeichnet, daß** die Tränkungslösung zu mehr als 90 Gew.-% - bezogen auf das Gesamtgewicht der Tränkungslösung - aus Wasser besteht.

7. Tücher nach einem der vorhergehenden Ansprüche, **dadurch gekennzeichnet, daß** die Tränkungslösung auf einer alkoholischen Basis beruht.

8. Tücher nach einem Ansprüche 3 bis 6, **dadurch gekennzeichnet, daß** die Tränkungslösung einen oder mehrere kosmetische oder dermatologische Hilfs-, Zusatz- und/oder Wirkstoffe enthält, gewählt aus der Gruppe: Moisturizer, Wachse, Konservierungsmittel, Antioxidantien, Farbstoffe, Pflanzenextrakte, UV-Filter, Pigmente, Deo- und Antitranspirant-Wirkstoffe, dermatologische Wirkstoffe sowie Parfüm.

## Claims

1. Cosmetic and dermatological towelettes, where the towelettes consist of a water-jet-compacted and water-jet-embossed nonwoven, which have been moistened with cosmetic and dermatological impregnation solutions which have a viscosity of less than 2000 mPa.s (measuring device: Haake Viskotester VT-02 at 25°C), **characterized in that** the thickness of the embossed nonwoven having the raised areas produced by the embossing is between 5% and 50% greater than the thickness of the unembossed nonwoven and a uniform sequence of raised areas and dips arises in the material.

2. Towelettes according to Claim 1, **characterized in that** the thickness of the embossed nonwoven having the raised areas produced by the embossing is between 10% and 25% greater than the thickness of the unembossed nonwoven.

3. Towelettes according to one of the preceding claims, **characterized in that** the weight ratio of the unimpregnated towelette to the impregnation solution is chosen from the range from 1:1 to 1:5.

4. Towelettes according to one of the preceding claims, **characterized in that** the impregnation solution consists of oils including silicone oils and/or lipophilic substances and/or comprises less than 0.5% by weight of water - based on the total weight of the impregnation solution.

5. Towelettes according to one of the preceding claims, **characterized in that**, besides one or more oil phases, the impregnation solution additionally comprises one or more water phases and is in the form of a W/O, O/W, W/O/W or O/W/O emulsion, a microemulsion, a Pickering emulsion, a sprayable emulsion or a hydrodispersion.

6. Towelettes according to one of the preceding claims, **characterized in that** the impregnation solution consists of more than 90% by weight - based on the total weight of the impregnation solution - of water.

7. Towelettes according to one of the preceding claims, **characterized in that** the impregnation solution is based on an alcohol.

8. Towelettes according to one of Claims 3 to 6, **characterized in that** the impregnation solution comprises one or more cosmetic or dermatological auxiliaries, additives and/or active ingredients chosen from the group: moisturizers, waxes, preservatives, antioxidants, dyes, plant extracts, UV filters, pigments, deodorant and antiperspirant active ingredients, dermatological active ingredients, and perfume.

## Revendications

1. Lingettes cosmétiques et dermatologiques, les lingettes étant constituées d'un non-tissé résistantes au jet d'eau et gaufrées sous jet d'eau, qui sont imprégnées de solutions d'imprégnation cosmétiques et dermatologiques ayant une viscosité de moins de 2 000 mPa.s (appareil de mesure viscosimètre Haake VT-02 à 25°C), **caractérisées en ce que** l'épaisseur du non-tissé gaufré avec les saillies produites par la gaufrage est d'entre 5 % et 50 % supérieure à l'épaisseur du non tissé non gaufré et une succession régulière de saillies et de creux est formée dans le matériau.

2. Lingettes selon la revendication 1, **caractérisées en ce que** l'épaisseur du non-tissé gaufré avec les saillies produites par le gaufrage est d'entre 10 % et 25 % supérieure à l'épaisseur du non tissé non gaufré.

3. Lingettes selon l'une quelconque des revendications précédentes, **caractérisées en ce que** le rapport pondéral de la lingette non imprégnée à la solution d'imprégnation est choisi dans la plage de 1:1 à 1:5.

4. Lingettes selon l'une quelconque des revendications précédentes, **caractérisées en ce que** la solution d'imprégnation consiste en des huiles, y compris des huiles de silicone, et/ou en des substances lipophiles et/ou contient moins de 0,5 % en poids d'eau - par rapport au poids total de la solution d'imprégnation.

5. Lingettes selon l'une quelconque des revendications précédentes, **caractérisées en ce que** la solution d'imprégnation, outre une ou plusieurs phases huileuses, contient additionnellement une ou plusieurs phases aqueuses et se trouve sous forme d'une émulsion E/H, H/E, E/H/E ou H/E/H, d'une microémulsion, d'une émulsion de Pickering, d'une émulsion pulvérisable ou d'une hydrodispersion.

6. Lingettes selon l'une quelconque des revendications précédentes, **caractérisées en ce que** la solution d'imprégnation est constituée d'eau à raison de plus de 90 % en poids - par rapport au poids total de la solution d'imprégnation.

7. Lingettes selon l'une quelconque des revendications précédentes, **caractérisées en ce que** la solution d'imprégnation repose sur une base alcoolique.

8. Lingettes selon l'une quelconque des revendications 3 à 6, **caractérisées en ce que** la solution d'imprégnation contient un ou plusieurs adjuvants, additifs et/ou actifs cosmétiques ou dermatologiques, choisis dans le groupe : hydratants, cires, conservateurs, antioxydants, colorants, extraits de plantes, filtres UV, pigments, actifs déodorants et antisudoraux, actifs dermatologiques ainsi que parfum.
